Europäisches Patentamt

European Patent Office

Office européen des brevets

(11) Publication number: **0 339 176**
**A1**

## EUROPEAN PATENT APPLICATION

(21) Application number: 89100019.2

(22) Date of filing: 02.01.89

(51) Int. Cl.4: **A61K 9/02** , **A61K 31/19**

The title of the invention has been amended (Guidelines for Examination in the EPO, A-III, 7.3).

(30) Priority: 16.02.88 GB 8803561

(43) Date of publication of application:
02.11.89 Bulletin 89/44

(84) Designated Contracting States:
AT BE CH DE ES FR GB GR IT LI NL SE .

(71) Applicant: AMERICAN CYANAMID COMPANY
1937 West Main Street P.O. Box 60
Stamford Connecticut 06904-0060(US)

(72) Inventor: Gould, Philip L.
14 Canterbury Road Stubbington
Fareham Hampshire(GB)
Inventor: Potts, Angela C.
7 Martin Avenue Stubbington
Fareham Hampshire(GB)

(74) Representative: Wächtershäuser, Günter, Dr.
Tal 29
D-8000 München 2(DE)

(54) Suppository containing a non-steroidal anti-inflammatory medicament.

(57) A suppository formulation comprises a suppository base and, as an active ingredient, (1,1'-biphenyl-4-acetic acid or a pharmaceutically acceptable salt thereof. The suppository formulation is useful for the treatment of such conditions as rheumatoid artiritis and osteoarthrosis.

EP 0 339 176 A1

## SUPPOSITORY

This invention relates to a suppository containing as active ingredient a non-steroidal anti-inflammatory drug.

Non-steroidal, anti-inflammatory drugs are commonly used in the treatment of such conditions as rheumatoid arthritis and osteoarthrosis. One such drug which is widely prescribed is Fenbufen, or 4-(biphenyl-4-yl)-4-oxo-butyric acid, which, when administered orally in doses of 600-900 mg daily, is well tolerated by the patient.

Rectal administration of non-steroidal anti-inflammatory drugs can be clinically advantageous in some circumstances, in that it can prolong the effect of the drug. For example, when certain non-steroidal drugs are given rectally to a patient immediately prior to retiring for the night, sufficient blood levels of the drug are still available some 6-7 hours later to prevent morning stiffness. However, in the case of Fenbufen, the high level required for the nocturnal dose (450-600 mg) makes it difficult to deliver this drug successfully from the rectum. Indeed, a clinical bioavailability study which we have conducted on Fenbufen has demonstrated low bioavailability of the active ingredient from suppositories, and all previous attempts to overcome this problem have failed. Accordingly, Fenbufen-containing suppository formulations for rectal administration have not been available, which is a disadvantage when Fenbufen would otherwise be the drug of choice for the patient.

The present invention represents a new approach to this problem.

Fenbufen is a so-called "pro-drug", that is the compound is not itself active but rather, it metabolizes in vivo, inter alia, to (1,1'-biphenyl)-4-acetic acid from which the activity of Fenbufen derives. However, this active metabolite is badly tolerated in the gastrointestinal tract and cannot be administered as such at therapeutic doses orally.

We have now discovered, in accordance with the present invention, that, unlike Fenbufen itself, (1,1'-biphenyl)-4-acetic acid is well absorbed rectally from a suppository formulation. Moreover on the basis of a study which we have made in dogs, it seems likely that, given rectally, (1,1'-biphenyl)-4-acetic acid will be better tolerated by the patient. Thus, there can be made available for the first time a suppository formulation which can be regarded as clinically equivalent to a Fenbufen-containing suppository without, however, encountering the hitherto unresolved problems of successfully formulating such a Fenbufen suppository.

Accordingly, the present invention broadly provides a suppository formulation comprising a suppository base and, as an active ingredient, (1,1'-biphenyl)-4-acetic acid or a pharmaceutically acceptable salt thereof.

Unlike Fenbufen suppositories, there are no underlying problems in the formulation of the suppositories of this invention and, for example, conventional suppository bases can be employed. Thus, we can use hydrophilic waxes, such as the polyethylene glycols (eg PEG 1500, PEG 3000, PEG 4000 and mixtures thereof) which dissolve in the rectal fluid; hydrophobic waxes, from which the active ingredient is released by partitioning into the rectal fluid following spreading and melting of the suppository base, such as cocoa butter; and semi-synthetic hydrophobic and hydrophilic waxes such as those sold under the trade names "Witepsol" (Dynamit Nobel Ab) or "Suppocire" (Gattefosse Etablissements). We currently prefer to use a semi-synthetic hydrophilic wax which includes an amphiphilic component for improved rectal absorption, for example "Suppocire AP" (Gattefosse).

Likewise, the incorporation of the (1,1'-biphenyl)-4-acetic acid into the suppository base may follow standard techniques well known to those skilled in the art. Also, if desired, minor amounts of conventional additives may also be included in the suppository formulation, for example viscosity modifying agents such as microcrystalline waxes, microcrystalline celluloses and lecithin; surfactants and counter-ion species to enhance rectal absorption such as salicylate salts; and materials to reduce rectal irritation such as fractionated coconut oils.

In the preferred embodiments of the suppository of the invention, a pharmaceutically acceptable salt of (1,1'-biphenyl)-4-acetic acid is used in order to enhance the solubility of the active ingredient in the small volume of rectal fluid found in vivo, and thereby give optimum conditions for complete drug absorption over a prolonged period. The sodium, ethanolamine and N-methylglucamine salts are especially preferred since they increase the solubility of (1,1'-biphenyl)-4-acetic acid in water by some 8-9 fold.

One feature and particular advantage, of using (1,1'-biphenyl)-4-acetic acid, rather than Fenbufen itself, is that only relatively low doses of the active ingredient are required. Thus, typically a single dose suppository in accordance with this invention will contain 30-100 mg, preferably about 50 mg of (1,1'-biphenyl)-4-acetic acid, or an equivalent amount of a pharmaceutically acceptable salt. The suppository itself may be of conventional size, for example the normal suppository size for adult dosing is 2g.

The suppository of this invention may be used in the treatment of the same clinical conditions as indicated for Fenbufen itself, and if desired may be used in conjunction with Fenbufen oral therapy.

The invention is illustrated by the Examples which follow:

## Example 1

A number of suppositories were prepared so as to contain 50 mg of (1,1'-biphenyl)-4-acetic acid active.

The suppositories were made by first melting the selected suppository base in a beaker on a water bath and then micronised (1,1'-biphenyl)-4-acetic acid, or its ethanolamine salt, were carefully added under constant stirring. Whilst the mixture of active ingredient and base was still molten, it was poured into plastics moulds (of 1 ml capacity) and then allowed to cool. The resulting suppositories, weighing approx. 1g., were then heat sealed using a Dott Bonapace BP6 heat sealer.

The ethanolamine salt of (1,1'-biphenyl)-4-acetic acid used in these experiments was made as follows. First, (1,1'-biphenyl)-4-acetic acid (230g) was suspended in methanol (1800 ml) and then a solution of ethanolamine (209g) in methanol (300 ml) was added. The resulting reaction solution was heated to 62°C and held at that temperature for 4 minutes, and then cooled. The solid product was collected by filtration, washed well with n-hexane and dried in vacuo. The colourless salt which resulted (mp 126-128°C) was found by analysis to be C70.45, H7.15, N5.13 (Theoretical valves: C70.33, H6.96, N5.13).

The compositions of the suppositories thus prepared are given below, wherein BPAA is used as an abbreviation for (1,1'-biphenyl)-4-acetic acid.

| Suppository A | % by weight |
|---|---|
| BPAA (micronised) | 5.263 |
| Witepsol H12* | 94.737 |
| | 100.000 |

*Witepsol H12 is a saturated fatty acid triglyceride of chain length C10-C18

| Suppository B | % by weight |
|---|---|
| BPAA-ethanolamine salt | 6.779 |
| Witepsol H12 | 93.221 |
| | 100.000 |

| Suppository C | % by weight |
|---|---|
| BPAA-ethanolamine salt | 5.60 |
| PEG 1500 | 94.40 |
| | 100.00 |

| Suppository D | % by weight |
|---|---|
| BPAA-ethanolamine salt (micronised) | 6.779 |
| Suppocire AP* | 93.221 |
| | 100.000 |

*Suppocire AP is composed of glycerides of $C_{12}-C_{18}$ polyoxyethylenes

## Example 2

To test the bioavailability of active ingredient from the suppository formulations A-D of Example 1, a series of experiments was performed on dogs.

Each animal was fasted for approximately 20 hours, and was then given a cleansing enema (150 ml of physiological saline at 37° C). The emptiness of the anal canal was verified by visual inspection. The test suppositories were then administered as a single dose to each animal by the rectal route. In each case, administration of the suppositories was at the rate of 5mg of BPAA active per kilogram of dog body weight.

Serum samples were periodically extracted from the test dogs over a time period of 72 hours and assayed for BPAA using a specific high performance reverse phase liquid chromatographic technique.

The results are presented in Table I below.

TABLE I

| Suppository Formulation | Concentration (mcg/ml) of BPAA in Serum* | | | | | | | | |
|---|---|---|---|---|---|---|---|---|---|
| | 0 hr | 1 hr | 2 hr | 3 hr | 5 hr | 7 hr | 24 hr | 48 hr | 72 hr |
| A | N.R | 19.7 | 28.2 | 29.1 | 22.5 | 18.6 | 10.2 | 3.6 | 1.7 |
| B | N.R | 27.9 | 30.6 | 28.6 | 21.4 | 16.9 | 10.4 | 3.05 | 0.72 |
| C | N.R | 20.5 | 28.5 | 24.1 | 22.9 | 18.8 | 9.4 | 2.5 | 0.8 |
| D | N.R | 24.6 | 31.0 | 31.8 | 25.4 | 23.2 | 13.7 | 5.7 | 2.2 |
| N.R = Not reportable (ie less than 0.1 mcg/ml) | | | | | | | | | |

* In each case, the serum concentration reported are the average values for four dogs.

From Table I above, it will be noted that in these dog studies, the active ingredient was well absorbed from each type of formulation, with absorption being rapid, with a time to peak serum concentration of about 2-3 hours in each case.

For dogs, the therapeutic concentration of (1,1'-biphenyl)-4-acetic acid in serum is from 10 g/ml (Chiccarelli et al, "Metabolic and Pharmacobinetic Studies with Fenbufen in Man", Arzneimittel-Forschung/Drug Research, 30 (I) 1980 728-735). Inspection of the data in Table I shows that for Suppositories A-D this minimum level was maintained for about 24 hours post-administration.

Suppository formulation D, using Suppocire AP as the base and the ethanolamine salt of the active ingredient, generally gave higher serum concentrations of active ingredient at all time periods than the other formulations.

COMPARATIVE EXAMPLE

In an experiment similar to that of Example 2, the bioavailability of BPAA from a Fenbufen-containing suppository was also tested on dogs.

The composition of the Fenbufen-containing suppository was as follows:

| | % by weight |
|---|---|
| Fenbufen | 15.79 |
| Suppocire AS2X | 84.21 |
| | 100.00 |

The suppository was made by the procedure described in Example 1. Suppocire AS2X is a semi-synthetic glyceride produced from hydrogenated vegetable oils by interesterification with ethoxylated fatty acid esters.

The suppositories were administered at the rate of 30 mg of Fenbufen per kilogram of dog body weight.

The concentrations (mcg/ml) of BPAA in serum at different time periods following administration were as follows, being average values for six dogs:

| Time (hours) | BPAA Concentrations in Serum (mcg/ml) |
|---|---|
| 1 | 6.3 |
| 2 | 8.7 |
| 4 | 8.1 |
| 7 | 6.65 |
| 9 | 8.4 |
| 24 | 6.3 |

From the above results it will be noted that the concentration of BPAA in the blood did not reach the therapeutically effective level of 10 mcg/ml even though the Fenbufen was administered at the high dosage level of 30 mg/kg of body weight. This study therefore illustrates the poor bioavailability of the active metabolite BPAA from Fenbufen-containing suppositories.

**Claims**

1. A suppository formulation comprising a suppository base and, as an active ingredient, (1,1'-biphenyl)-4-acetic acid or a pharmaceutically acceptable salt thereof.

2. A formulation according to Claim 1, wherein said salt is the sodium, ethanolamine or N-methylglucamine salt.

3. A formulation according to Claim 1 or Claim 2, containing form 30-100 mg of (1,1'-biphenyl)-4-acetic acid.

4. A formulation according to any preceding claim, wherein the suppository base is of amphiophilic character.

5. A suppository formulation substantially as described in Example 1 herein.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 106, no. 16, April 1987, page 390, abstract no. 125779z, Columbus, Ohio, US; U. KANETO et al.: "Possible utility of beta-cyclodextrin complexation in the preparation of biphenyl acetic acid supository", & YAKUGAKU ZASSHI 1986, 106(12), 1126-30 * Abstract * | 1 | A 61 K 9/02<br>A 61 K 31/19 |
| Y | IDEM | 2-5 | |
| Y | JOURNAL OF MEDICINAL CHEMISTRY, vol. 25, no. 4, April 1982, pages 446-451, The American Chemical Society, Washington, D.C., US; D.A. WALSH et al.: "Antiinflammatory agents. 2. Syntheses and antiinflammatory activity of substituted 2-Aminophenylacetic acid derivatives * Abstract * | 1,2 | |
| Y | CHEMICAL ABSTRACTS, vol. 100, no. 18, April 1984, page 355, abstract no. 144881u, Columbus, Ohio, US; A. SALVADO et al.: "In vitro release of paracetamol in suppositories and bioavailability in the rabbit", & CIENC. IND. FARM. 1983, 2(10), 339-44 * Abstract * | 4-5 | TECHNICAL FIELDS SEARCHED (Int. Cl.4)<br><br>A 61 K 9/00<br>A 61 K 31/00 |
| A | US-A-3 784 704 (E. COHEN et al.) * Abstract; column 8, lines 10-45; claim 1 * | 3 | |
| | -/- | | |

The present search report has been drawn up for all claims

| Place of search | Date of completion of the search | Examiner |
|---|---|---|
| THE HAGUE | 28-04-1989 | DULLAART A.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | J.E.F. REYNOLDS et al.: "Martindale, the extra pharmacopoeia", 28th edition, 1982, page 252, no. 2646-f, The Pharmaceutical Press, London, GB<br>* Column 2: "Fenbufen" - column 3, line 9 * | 1-3 | |

TECHNICAL FIELDS
SEARCHED (Int. Cl.4)

The present search report has been drawn up for all claims

| Place of search | Date of completion of the search | Examiner |
|---|---|---|
| THE HAGUE | 28-04-1989 | DULLAART A.W.M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)